(19) Europäisches Patentamt
European Patent Office
Office européen des brevets

(11) Veröffentlichungsnummer: **0 028 703**
B1

(12) **EUROPÄISCHE PATENTSCHRIFT**

(45) Veröffentlichungstag der Patentschrift: **17.11.82**

(51) Int. Cl.³: **C 07 C 49/04,** C 07 C 45/75, C 07 C 49/76, C 07 C 49/403 // B01J27/18

(21) Anmeldenummer: **80105993.2**

(22) Anmeldetag: **03.10.80**

(54) **Verfahren zur Herstellung von alpha-methylsubstituierten Carbonylverbindungen.**

(30) Priorität: **12.10.79 DE 2941386**

(43) Veröffentlichungstag der Anmeldung: **20.05.81 Patentblatt 81/20**

(45) Bekanntmachung des Hinweises auf die Patenterteilung: **17.11.82 Patentblatt 82/46**

(84) Benannte Vertragsstaaten: **BE CH DE FR GB LI NL**

(56) Entgegenhaltungen:
**DE-A-2 326 406**
**DE-B-1 922 755**
**US-A-3 410 909**

(73) Patentinhaber: **BASF Aktiengesellschaft, Carl-Bosch-Strasse 38, D-6700 Ludwigshafen (DE)**

(72) Erfinder: **Heilen, Gerd, Dr., Schifferstadter Strasse 1B, D-6720 Speyer (DE)**
Erfinder: **Halbritter, Klaus, Dr., Schwarzwaldstrasse 19, D-6800 Mannheim 1 (DE)**
Erfinder: **Gramlich, Walter, Dr., Auf der Hoehe 11, D-6803 Edingen (DE)**

**EP 0 028 703 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung von $\alpha$-methylsubstituierten Carbonylverbindungen

Die Erfindung betrifft ein Verfahren zur Herstellung $\alpha$-methylsubstituierter Ketone durch Umsetzen der entsprechenden nicht methylsubstituierten Ketone mit Formaldehyd unter hydrierenden Bedingungen.

Die Einführung von Methylgruppen in $\alpha$-Stellung zur Carbonylgruppe ist an sich bekannt. So ist beispielsweise in »Houben-Weyl, Methoden der organischen Chemie, Bd. VII, 2b, S. 1385 ff« beschrieben, Carbonylverbindungen durch Umsetzen mit Alkylhalogeniden — bevorzugt den Bromiden oder Jodiden — zu alkylieren. Nachteilig an dieser Methode ist, daß man die relativ teuren Alkylhalogenide als Ausgangsverbindungen verwenden muß sowie die Verwendung meist stöchiometrischer Mengen an alkalischen Substanzen, die oft nur in Kombination mit aufwendigen Lösungsmitteln, wie Alkaliamid in flüssigem $NH_3$, eingesetzt werden können.

Eine weitere bekannte Methode besteht in der Aldolkondensation von Carbonylverbindungen mit Formaldehyd, Dehydratisierung der gebildeten Aldoladdukte und anschließender Hydrierung der $\alpha,\beta$-ungesättigten Carbonylverbindung zum gewünschten Endprodukt. Hier wird zwar mit Formaldehyd ein preiswertes Ausgangsmaterial verwendet, die Reaktion muß jedoch in zwei bzw. drei getrennten Stufen in verschiedenen Apparaten durchgeführt werden.

Eine einstufige Alkylierung wird in der DE 2 257 675 beschrieben. Hiernach lassen sich Ketone an einem Kupfer- bzw. Silberkatalysator mit Methanol zu den entsprechenden $\alpha$-methylsubstituierten Ketonen umsetzen. Nachteile dieses Verfahrens sind die Bildung von zwei- oder mehrfach alkylierten Verbindungen sowie die z. T. wenig guten Ausbeuten.

So entstehen z. B. bei der Umsetzung von Methyläthylketon neben Methylisopropylketon in vergleichbarer Menge auch Äthylisopropylketon sowie Diäthylketon. Darüber hinaus bildet sich sogar ein gewisser Anteil an Diisopropylketon.

Aus der DE-AS 1 922 755 ist es ferner bekannt, durch Eigenkondensation von Ketonen neue Ketone mit doppelter Anzahl von C-Atomen herzustellen, z. B. Methylisobutylketon aus Aceton. Als Katalysator werden hierfür Phosphate von Zr, Hf, Ti oder Sn verwendet, welche zusätzlich geringe Mengen an Palladium enthalten.

Es wurde nun überraschend gefunden, daß diese Katalysatoren auch für die Umsetzung von Carbonylverbindungen mit Formaldehyd geeignet sind. Dieses war nicht zu erwarten, da davon ausgegangen werden mußte, daß bei Verwendung dieser Katalysatoren bevorzugt eine Eigenkondensation der eingesetzten Carbonylverbindungen eintritt. Wie sich jedoch zeigte, tritt bei der gemischten Kondensation von Carbonylverbindungen mit Formaldehyd an diesen Katalysatoren auch bei Verwendung eines großen Überschusses an der Carbonylverbindung diese Eigenkondensation nur in zu vernachlässigendem Ausmaß auf.

Gegenstand der Erfindung ist dementsprechend ein Verfahren zur Herstellung von Carbonylverbindungen der allgemeinen Formel I

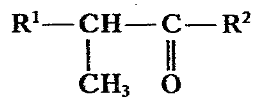

(I)

in der $R^1$ und $R^2$ eine geradkettige oder verzweigte Alkylgruppe mit 1 bis 8, vorzugsweise 1 bis 4 C-Atomen, eine Arylgruppe oder eine Aralkylgruppe bedeuten,
$R^1$ zusätzlich H bedeuten kann und
$R^1$ und $R^2$ zusammen mit den zwischen ihnen liegenden C-Atomen auch Glieder eines alicyclischen Ringes bedeuten können,
dadurch gekennzeichnet, daß man ein Keton der allgemeinen Formel II

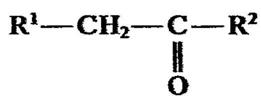

(II)

bei Temperaturen von 50–230°C in Gegenwart von Wasserstoff und einem Katalysator, der als wirksame Bestandteile

a) ein Phosphat von mindestens einem der Metalle Zirkon, Titan, Hafnium und Zinn und
b) metallisches Palladium auf diesem Phosphat abgeschieden enthält,

mit Formaldehyd umsetzt.

Die als Ausgangsverbindungen benötigten Ketone der Formel II sind bekannte, handelsübliche Verbindungen. Genannt seien beispielsweise Aceton, Methyläthylketon, Diäthylketon, Cyclohexanon, Acetophenon und 4-Phenyl-butan-2-on.

Die in dem erfindungsgemäßen Verfahren verwendeten Katalysatoren sind aus der DE-AS 1 922 755 bekannt. Sie bestehen im wesentlichen aus einem Phosphat der Elemente Zirkon, Hafnium, Titan oder Zinn und enthalten zusätzlich metallisches Palladium. Das Molverhältnis $PO_4^{3-}$ : Metall unterliegt keiner Beschränkung. Aus Gründen der Wirtschaftlichkeit wendet man im allgemeinen solche Metallphosphate, in denen einer der Bestandteile nicht die dreifache molare Menge des anderen überschreitet. Besonders vorteilhaft sind Katalysatoren, in denen das Molverhältnis $PO_4^{3-}$ : Metall = 0,6 bis 1,7 : 1 ist. Die Menge an metallischem Palladium ist dabei nicht kritisch und wird im wesentlichen durch wirtschaftliche Gesichtspunkte bestimmt. Sie beträgt im allgemeinen 0,1 bis 5,3 Gew.-%, insbesondere 0,2 bis 3,0 Gew.-%, bezogen auf das Metallphosphat. Die Herstellung der Katalysatoren erfolgt in an sich bekannter Weise und

ist in der oben genannten DE-AS sehr ausführlich beschrieben, so daß sich nähere Ausführungen hierüber erübrigen. Die Katalysatoren können sowohl in pulverisierter Form wie auch in Form von Kugeln, Strängen, Tabletten oder als Granulat eingesetzt werden.

Formaldehyd kann in verschiedenen Formen in die Reaktion eingebracht werden. Möglich ist z. B. die Verwendung von Paraformaldehyd, gelöst oder suspendiert in der umzusetzenden Carbonylverbindung wie auch der Einsatz von handelsüblichen Formaldehyd-Lösungen, wie einer 30–40%igen Lösung von Formaldehyd in Wasser oder in niederen Alkoholen.

Das molare Verhältnis von umzusetzender Carbonylverbindung der Formel II zu Formaldehyd ist nicht kritisch. Besonders gute Ergebnisse werden erhalten, wenn die umzusetzende Carbonylverbindung im Überschuß angewendet wird. Der molare angewendete Überschuß kann dabei im Verhältnis von 1 : 1 bis 20 : 1 variieren. Ein höherer Überschuß hat im allgemeinen keine Vorteile und verbietet sich daher aus wirtschaftlichen Gründen. Unter diesen Überlegungen ist ein Überschuß von 1 : 1 bis 15 : 1 besonders günstig. Bemerkenswerterweise findet indes auch bei höherem Überschuß der Carbonylverbindung eine Eigenkondensation nur in geringem Maße statt.

Das erfindungsgemäße Verfahren kann sowohl kontinuierlich als auch diskontinuierlich durchgeführt werden. Für den kontinuierlichen technischen Betrieb empfiehlt es sich aus verfahrenstechnischen Gründen, den Katalysator in einer Reaktorsäule fest anzuordnen und das Reaktionsgemisch sowie Wasserstoff über ein derartiges Festbett zu leiten. Für den diskontinuierlichen Betrieb wird der Katalysator zweckmäßigerweise in suspendierter Form verwendet.

Das Verfahren gelingt prinzipiell bereits bei Normaldruck, zur Erhöhung der Reaktionsgeschwindigkeit ist jedoch — insbesondere bei Einsatz niedrigsiedender Carbonylverbindungen — die Anwendung von erhöhtem Druck zweckmäßig. Üblicherweise wendet man einen Wasserstoffdruck von bis zu 100 bar an. Noch höhere Drücke bringen im allgemeinen keine Vorteile und sind daher nicht wirtschaftlich. Die wirtschaftlich günstigsten Ergebnisse werden bei Drücken im Bereich von 1–50 bar erreicht.

Für die Reaktionstemperatur empfiehlt sich ein Bereich von 50–230°C, vorzugsweise 70–210°C. Bei Temperaturen unter 50°C sinkt die Reaktionstemperatur merklich ab, und bei Temperaturen über 230°C werden Ausbeuteverluste durch fortschreitende Aldolkondensationen beobachtet.

Sofern die umzusetzende Carbonylverbindung flüssig ist, ist die Verwendung eines Lösungsmittels nicht erforderlich. Feste Carbonylverbindungen können in allen unter den Reaktionsbedingungen inerten Flüssigkeiten gelöst werden, beispielsweise in Paraffinen, wie Pentan, Hexan und Octan; Cycloparaffinen, wie Cyclohexan; Aromaten, wie Benzol und Toluol; Alkoholen, wie Methanol, Äthanol und Isopropanol; oder Estern, wie Essigsäuremethyl- oder -äthylester.

Mit Hilfe des erfindungsgemäßen Verfahrens können die größtenteils als wertvolle Zwischenprodukte für die Herstellung von Farbstoffen, Pflanzenschutzmitteln oder Arzneimitteln begehrten $\alpha$-methylsubstituierten Carbonylverbindungen auf technisch einfache und vorteilhafte Weise hergestellt werden.

## Beispiel 1

Ein gemäß der DE-AS 1 922 755 (vgl. Tabelle 1) durch Fällung mit Ammoniakwasser aus einer wäßrigen Lösung entsprechender Mengen an $ZrOCl_2 \cdot 8\,H_2O$, $PdCl_2$ und $H_3PO_4$ hergestellter Katalysator, bestehend aus Zirkonphosphat und einem Zusatz von 0,5 Gew.-% metallischem Palladium in Pillenform (Ø 3 mm) wurde in einen Rohrreaktor von 1,8 l Inhalt und einem inneren Durchmesser von 4,5 cm gefüllt. Durch diesen Rohrreaktor, der unter einem Wasserstoffdruck von 30 bar stand, wurden bei 180°C stündlich 2,5 l eines Gemisches geleitet, welches zu 87,8% aus Methyläthylketon und zu 12,2% aus einer wäßrigen Formaldehyd-Lösung (30 Gew.-% Formaldehyd) bestand.

Der unter diesen Bedingungen anfallende Reaktionsaustrag wurde gesammelt und fraktionierend aufdestilliert; die erhaltenen Fraktionen wurden gaschromatographisch analysiert. Danach zeigte der Reaktionsaustrag (ohne Berücksichtigung des eingesetzten bzw. gebildeten Wassers) die folgende Zusammensetzung:

unumgesetztes

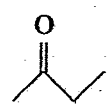 85,6 Gew.-%

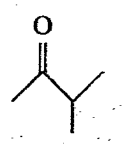 9,7 Gew.-%

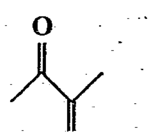 0,8 Gew.-%

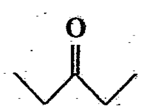 1,0 Gew.-%

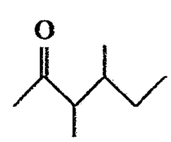 0,1 Gew.-%

Dies entspricht einer Ausbeute an Methylisopropylketon von 82,9%, bezogen auf eingesetzten Formaldehyd. Bei zusätzlicher Berücksichtigung des Methylisopropenylketons (Vorstufe für Methylisopropylketon) erhöht sich die Ausbeute auf 89,7%. Bemerkenswert ist der praktisch zu vernachlässigende Anteil an gebildetem 2,3-Dimethylhexan-2-on, dem Eigenkondensationsprodukt des Methyläthylketons.

Beispiel 2

In der in Beispiel 1 beschriebenen Apparatur und am gleichen Katalysator wie im Beispiel 1 wurde bei 170°C und 30 bar $H_2$-Druck pro Stunde 1,0 l eines Gemisches umgesetzt, welches aus 82,5% Methyläthylketon und 17,5% einer wäßrigen Formaldehyd-Lösung mit einem Gehalt von 30% Formaldehyd bestand. Bei der Umsetzung dieses Zulaufgemisches wurde eine Ausbeute an Methylisopropylketon von 77,7%, bezogen auf eingesetzten Formaldehyd, erhalten. Bei Berücksichtigung des angefallenen Methylisopropenylketons als Wertprodukt steigt die Ausbeute auf 84,1%. Der Anteil an gebildetem 2,3-Dimethylhexan-2-on lag unter diesen Bedingungen bei 0,2%.

Beispiel 3

Ebenfalls in der in Beispiel 1 beschriebenen Apparatur und auf dem in Beispiel 1 beschriebenen Katalysator wurden bei 30 bar $H_2$-Druck stündlich 0,4 l eines Gemisches aus 68,4% Methyläthylketon und 31,6% einer wäßrigen 30%igen Formaldehyd-Lösung umgesetzt. Bei einer Reaktionstemperatur von 190°C bildete sich Methylisopropylketon in einer Ausbeute von 78,9%, bezogen auf eingesetzten Formaldehyd. Bei entsprechender Berücksichtigung des Methylisopropenylketons erhöht sich dieser Wert auf 83,2%. Auch hier lag der Anteil des gebildeten 2,3-Dimethyl-hexan-2-ons bei 0,1%.

Beispiel 4

In dem in Beispiel 1 beschriebenen Reaktor wurde an einem Kontakt, der aus Titanphosphat mit 0,5% Pd bestand, bei 160°C und 40 bar $H_2$-Druck stündlich 1,0 l eines Gemisches aus Aceton und wäßriger 30%iger Formaldehyd-Lösung umgesetzt. Das molare Verhältnis Aceton : Formaldehyd betrug 8 : 1. Bezogen auf den eingesetzten Formaldehyd wurde Methyläthylketon in einer Ausbeute von 73,3% erhalten. Der Anteil an gebildetem Methylisobutylketon, dem Dimerisierungsprodukt des Acetons, lag bei 0,8%.

Beispiel 5

In der in Beispiel 1 beschriebenen Apparatur und an dem in Beispiel 1 beschriebenen Kontakt wurden pro Stunde 0,5 l eines Gemisches aus Acetophenon und einer 30%igen isobutanolischen Formaldehydlösung bei 180°C und 30 bar $H_2$-Druck umgesetzt. Das molare Verhältnis zwischen Acetophenon und Formaldehyd betrug 8 : 1. Bezogen auf eingesetzten Formaldehyd wurde Propiophenon in einer Ausbeute von 52,8% erhalten.

Beispiel 6

In der in Beispiel 1 beschriebenen Apparatur und dem in Beispiel 1 beschriebenen Kontakt wurden pro Stunde 0,5 l eines Gemisches aus Cyclohexanon und einer 30%igen isobutanolischen Formaldehydlösung umgesetzt. Das molare Verhältnis zwischen Cyclohexanon und Formaldehyd betrug 6 : 1. Es wurde bei einer Temperatur von 180°C und einem Wasserstoffdruck von 30 bar gearbeitet. Das gewünschte 2-Methyl-cyclohexanon wurde in 45,3%iger Ausbeute, bezogen auf eingesetzten Formaldehyd, erhalten.

**Patentanspruch**

Verfahren zur Herstellung von Carbonylverbindungen der allgemeinen Formel I

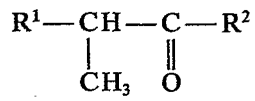
(I)

in der $R^1$ und $R^2$ eine geradkettige oder verzweigte Alkylgruppe mit 1 bis 8 C-Atomen, eine Arylgruppe oder eine Aralkylgruppe bedeuten,
$R^1$ zusätzlich H bedeuten kann und
$R^1$ und $R^2$ zusammen mit den zwischen ihnen liegenden C-Atomen auch Glieder eines alicyclischen Ringes bedeuten können,
dadurch gekennzeichnet, daß man ein Keton der allgemeinen Formel II

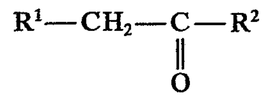
(II)

bei Temperaturen von 50–230°C in Gegenwart von Wasserstoff und einem Katalysator, der als wirksame Bestandteile

a) ein Phosphat von mindestens einem der Metalle Zirkon, Titan, Hafnium und Zinn und
b) metallisches Palladium auf diesem Phosphat abgeschieden enthält,

mit Formaldehyd umsetzt.

**Claim**

A process for the preparation of a carbonyl

compound of the general formula I

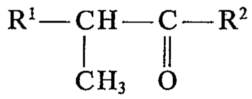 (I)

where $R^1$ und $R^2$ are straight-chain or branched alkyl of 1 to 8 carbon atoms or are aryl or aralkyl, $R^1$ may also be H, and $R^1$ and $R^2$ together with the carbon atoms between them may also be members of an alicyclic ring, characterized in that a ketone fo the general formula II

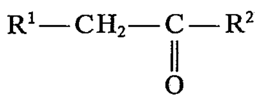 (II)

is reacted with formaldehyde at 50 – 230° C in the presence of hydrogen and of a catalyst which contains, as the active constituents,

a) a phosphate of at least one of the metals zirconium, titanium, hafnium and tin, and
b) metallic palladium deposited on this phosphate.

**Revendication**

Procédé de préparation de composés carbonylés de la formule générale I

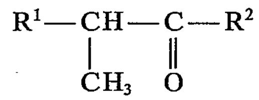 (I)

dans laquelle $R^1$ et $R^2$ désignent chacun un radical alcoyle en $C_1$ à $C_8$ à chaîne droite ou ramifiée ou un groupe aryle ou un groupe aralcoyle,
$R^1$ pouvant en outre désigner un atome d'hydrogène et
$R^1$ et $R^2$ pouvant constituer avec les atomes de carbone intermédiaires des chaînons d'un noyau alicyclique,
caractérisé en ce que l'on fait réagir une cétone de la formule générale II

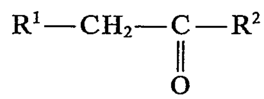 (II)

avec du formaldéhyde à des températures de 50 à 230°C en présence d'hydrogène et d'un catalyseur, qui contient comme composants actifs

a) un phosphate d'au moins un des métaux zirconium, titane, hafnium et étain et
b) du palladium métallique déposé sur ce phosphate.